# EUROPEAN PATENT APPLICATION

(11) **EP 4 317 403 A1**
(43) Date of publication of application: **07.02.2024**
(21) Application number: 22775160.9
(22) Date of filing: 10.03.2022
(51) Int. Cl.: C12M 1/34, G06T 7/00, G06T 7/60, C12Q 1/06, G01N 33/48, G01N 33/483

(54) **CELL COUNTING METHOD, CONSTRUCTION METHOD OF MACHINE LEARNING MODEL FOR CELL COUNTING, COMPUTER PROGRAM, AND STORAGE MEDIUM**

(30) Priority: 23.03.2021 JP 2021048521
(71) Applicant: SCREEN Holdings Co., Ltd., Kyoto-shi, Kyoto 602-8585 (JP)
(72) Inventor: TOKISUE, Shogo, Kyoto-shi, Kyoto 602-8585 (JP); OKAMOTO, Satoshi, Kyoto-shi, Kyoto 602-8585 (JP)
(74) Representative: Goddar, Heinz J.
(86) International application number: PCT/JP2022/010644
(87) International publication number: WO 2022/202368

(57) **Abstract**

A cell counting method according to this invention includes obtaining an image stack including a plurality of images of the cell mass obtained by bright-field imaging at mutually different depths of focus, generating a plurality of heat maps respectively corresponding to the plurality of images using a machine learning model, detecting a peak from each of the heat maps, associating the peaks belonging to mutually different ones of the heat maps and having a distance in a direction along a map plane and a distance in a direction of the depth of focus, the distances being respectively smaller than predetermined values, and counting number of the peaks while regarding a plurality of the associated peaks as one peak. The machine learning model is constructed by machine learning using sets of a teacher image and a ground truth image as teacher data, the teacher image being a bright-field image of a cell, the ground truth image being a heat map to which a weight increasing toward a central part of the cell is given.

## Description

### [Technical Field]

This invention relates to a technique for analyzing images obtained by imaging a cell mass and automatically counting the number of cells constituting the cell mass.

### [Background Art]

In the technical fields of pathological medicine and cell culture, the number of cells present in images obtained by imaging a cell mass composed of a plurality of cells is often used as a numerical criterion for judging a state, e.g. a developmental status, of cells or a tissue. It is a large burden on an operator to visually count a multitude of cells. For this reason, a technique for analyzing images and automatically counting the number of cells has been proposed.

For example, in a technique described in PTL 1, the shapes of observation objects are grasped from the brightness of an image obtained by microscopic imaging a specimen stained by a staining method for staining cell nuclei. When micronuclei are detected, the number of cells is obtained by counting the nuclei after association according to distances to main nuclei. Further, for example, in a technique described in PTL 2, an image of a specimen having a fluorescent label attached thereto is used to distinguish cells close to each other and having unclear boundaries. Specifically, a score of a target pixel is calculated from pixel values of a plurality of pixels in a region including the target pixel, and the positions or number of the individual cells are/is obtained based on that score.

### [Citation List]

### [Patent Literature]

[PTL 1] JP2001-269195A
[PTL 2] WO2005/057496

### [Summary of Invention]

### [Technical Problem]

If a cell mass to be observed has a three-dimensional structure and a plurality of cells overlap in a depth direction of an image, it is difficult to separate and count the individual cells by the method based on a luminance value of an image like the conventional technique described above. For example, in the observation of a fertilized egg (embryo), a multitude of cells are dense spherically and the individual cells are nearly transparent. Thus, it is very difficult to separate and individually count those cells from a luminance value of a single microscope image captured by receiving transmitted light or reflected light.

For this reason, it is required to establish a technique capable of more accurately obtaining the number of cells constituting a cell mass having a three-dimensional structure. Further, in the above conventional technique, since staining is applied or fluorescent labels are attached to the cells, application to the counting of living cells is not possible. Thus, it is more preferable to enable counting even if an image not dependent on an invasive imaging method is used.

### [Solution to Problem]

This invention was developed in view of the above problem and aims to provide a technique capable of automatically and accurately counting the number of cells from images obtained by imaging a cell mass.

To achieve the above object, one aspect of this invention is directed to a cell counting method for counting number of cells constituting a cell mass, the cell counting method including obtaining an image stack including a plurality of images of the cell mass obtained by bright field imaging at mutually different depths of focus, generating a plurality of heat maps respectively corresponding to the plurality of images using a machine learning model, detecting a peak from each of the heat maps, associating the peaks belonging to mutually different ones of the heat maps and having a distance in a direction along a map plane and a distance in a direction of the depth of focus, the distances being respectively smaller than predetermined values determined in advance, with each other, and counting number of the peaks, regarding a plurality of the associated peaks as one peak. Here, the machine learning model is constructed by machine learning, using sets of a teacher image and a ground truth image as teacher data, the teacher image being a bright-field image of a cell, the ground truth image being a heat map to which a weight larger inside a contour of the cell focused in the teacher image than outside the contour and increasing toward a central part of the cell is given.

In the invention thus configured, the number of the cells is counted based on a so-called multi-focus image stack obtained by imaging the cell mass in the bright field at mutually different depths of focus a plurality of times. In the bright-field imaging of a cell mass composed of nearly transparent cells, not only the cells appearing on the surface of the cell mass, but also the cells behind the former cells are expected to be imaged in a focused state in any one of the plurality of images by performing imaging at different depths of focus. Therefore, if the focused cell is extracted from each image, those cells are thought to cover the entire cells constituting the cell mass.

However, there is a possibility that one cell is focused in a plurality of images due to a relationship of a depth of field of an imaging optical system, a depth-of-focus change pitch during imaging, a cell size and the like. Thus, by merely counting the cells detected from each image, the cells may be repeatedly counted in some cases and a correct count result cannot be obtained. In the invention, this problem is solved by the following configuration.

Firstly, the cell in each image is specified by a method for detecting a peak from the heat map output by the machine learning model. The closer it is to the center of the cell, the larger weight is given to the heat map, and a peak position corresponds to a center position of the cell. Secondly, the number of the peaks is counted, regarding the peaks close to each other as one peak, out of the peaks detected from each image.

If cell regions detected from a plurality of the images having different depths of focus overlap each other, it is difficult to judge whether those cell regions correspond to the same cell or different cells only by specifying a region occupied by the cell in each image. On the other hand, for the purpose of obtaining the number of the cells, it is not an essential requirement to precisely specify the regions occupied by the cells in the images. Rather, a method for specifying the center position of the cell is suitable. This is because it can be judged whether or not the regions are derived from the same cell from a distance between the centers of the detected cells.

Specifically, if the cell size is known to a certain extent, a lower limit of the possible distance between those centers can be estimated. Accordingly, out of the peaks corresponding to the cell centers detected in the plurality of images, those at positions closer than the lower limit estimated in that way can be regarded as derived from the same cell. On the other hand, each of the isolated peaks can be thought to correspond to one cell. If all of a plurality of peaks at positions closer than a certain distance are regarded as one peak in this way, the number of the cells can be expressed by the number of the peaks. In this way, it is avoided that the same cell is repeatedly counted.

Further, the individual cell is detected by a method for estimating the center position of the cell, using the machine learning model constructed using the images including the focused images as the teacher images. If machine learning is performed, using images of cells not using an invasive method such as the staining of cell nuclei as teacher images, images of the cell mass serving as an object of counting captured in a non-invasive manner can be used.

Further, another aspect of this invention is direction to a machine learning model construction method for cell counting, the machine learning model construction method including obtaining a plurality of teacher images which are bright-field images of a cell mass, generating a ground truth image for each of the teacher images which is a pseudo heat map to which a weight is given, the weight being larger inside a contour of a cell focused in the teacher image than outside the contour and increasing toward a central part of the cell, and constructing a machine learning model by performing machine learning, using sets of the teacher image and the ground truth image as teacher data. In the invention thus configured, the machine learning model suitable to perform the above cell counting method can be efficiently constructed.

Further, another aspect of this invention is directed to a computer program for causing a computer to perform each step of the above cell counting method or machine learning model construction method for cell counting. Further, another aspect of this invention is directed to a computer-readable recording medium temporarily recording the computer program. In the invention thus configured, an existing computer device can be, for example, used as an execution subject of the invention.

### [Advantageous Effects of Invention]

As described above, according to the invention, a heat map having a weight given thereto, the weight increasing toward a center of a cell, is generated from each image of a multi-focus image stack. Peaks of the heat map are handled as centers of the cells and a plurality of peaks having a peak-to-peak distance smaller than a predetermined value are regarded as derived from the same cell and counted. Thus, even if a cell mass has a three-dimensional structure, the number of cells constituting the cell mass can be automatically and accurately counted.

The above and further objects and novel features of the invention will more fully appear from the following detailed description when the same is read in connection with the accompanying drawing. It is to be expressly understood, however, that the drawing is for purpose of illustration only and is not intended as a definition of the limits of the invention.

### [Brief Description of Drawings]

[FIG. 1] FIG. 1 is a diagram showing a schematic configuration of an imaging apparatus capable of performing a cell counting method according to the invention.
[FIG. 2A] FIG. 2A is a diagram schematically showing the structure of an embryo as a biological specimen in this embodiment.
[FIG. 2B] FIG. 2B is a diagram schematically showing the structure of an embryo as a biological specimen in this embodiment.
[FIG. 3] FIG. 3 is a flow chart summarizing the cell counting process of this embodiment.
[FIG. 4] FIG. 4 is a flow chart showing a process of constructing the machine learning model.
[FIG. 5A] FIG. 5A is a chart illustrating a process of generating the heat map.
[FIG. 5B] FIG. 5B is a chart illustrating a process of generating the heat map.
[FIG. 6] FIG. 6 is a diagram showing an example of the method for detecting the peak points.
[FIG. 7A] FIG. 7A is a diagram showing a process of consolidating the peak points.
[FIG. 7B] FIG. 7B is a diagram showing a process of consolidating the peak points.
[FIG. 8] FIG. 8 is a flow chart showing a first aspect of a proximate peak consolidation process.
[FIG. 9A] FIG. 9Ais a diagram schematically showing an example of this proximate peak consolidation process.
[FIG. 9B] FIG. 9B is a diagram schematically showing an example of this proximate peak consolidation process.
[FIG. 10] FIG. 10 is a flow chart showing a second aspect of the proximate peak consolidation process.
[FIG. 11] FIG. 11 is a diagram schematically showing an example of this proximate peak consolidation process.

### [Description of Embodiments]

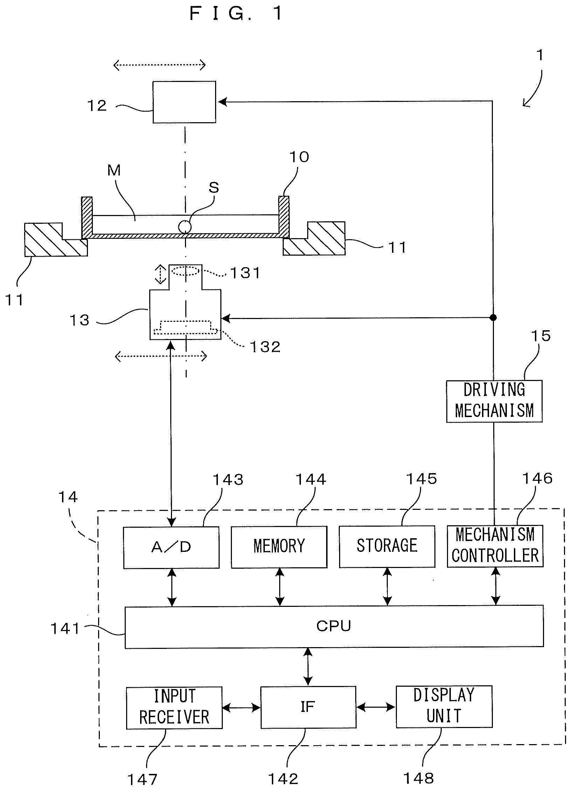

FIG. 1 is a diagram showing a schematic configuration of an imaging apparatus capable of performing a cell counting method according to the invention. This imaging apparatus 1 is an apparatus for imaging a biological specimen S such as cells C carried in a specimen container 10. The specimen container 10 may be called a dish and has a flat shape and an open upper surface. A predetermined amount of a liquid serving as a culture medium M is injected into the specimen container 10. The cells or the like cultured under predetermined culture conditions in this liquid serve as an imaging object of this imaging apparatus 1. The culture medium may be added with an appropriate reagent or may be gelled after being injected in a liquid state into the specimen container 10.

Note that a case where a biological specimen is used as the imaging object is illustrated here, the imaging object of the invention is not limited to this. For example, a tissue section, a pathological specimen or the like carried in an appropriate carrier may be the imaging object. Further, the specimen container may be a well plate provided with a plurality of recesses referred as "well" and a biological specimen carried in each well may be the imaging object.

The imaging apparatus 1 includes a holder 11 which holds the specimen container 10, an illuminator 12 arranged above the holder 11, an imager 13 arranged below the holder 11 and a controller 14 which includes a CPU 141 controlling the operation of these components. The holder 11 holds the specimen container 10 in a substantially horizontal posture by being held in contact with a peripheral edge part of the lower surface of the specimen container 10.

The illuminator 12 emits an illumination light toward the specimen container 10 held by the holder 11. For example, a white LED (light emitting diode) may be used as a light source of the illumination light. A combination of the light source and an appropriate illumination optical system are used as the illuminator 12. The imaging object in the specimen container 10 is illuminated by the illuminator 12 from above.

The imager 13 is provided below the specimen container 10 held by the holder 11. In the imager 13, an imaging optical system is arranged at a position right below the specimen container 10. An optical axis of the imaging optical system extends in a vertical direction. FIG. 1 shows a side view. An up and down direction of the figure indicates a vertical direction.

By the imager 13, a bright-field imaging of the imaging object in the specimen container 10 is performed as follows. Light emitted from the illuminator 12 and incident on the surface of the liquid from above the specimen container 10 illuminates the imaging object. Light transmitted downward from the bottom surface of the specimen container 10 is incident to a light receiving surface of an imaging element 132 via the imaging optical system of the imager 13 including an objective lens 131. An image (bright-field image) of the imaging object formed on the light receiving surface of the imaging element 132 by the imaging optical system is imaged by the imaging element 132. The imaging element 132 is an area image sensor having a two-dimensional light receiving surface. A CCD sensor or a CMOS sensor can be used as the imaging element 132.

The imager 13 is capable of moving in the horizontal direction and the vertical direction by a mechanism controller 146 provided in the controller 14. Specifically, the mechanism controller 146 moves the imager 13 in the horizontal direction by operating a driving mechanism 15 based on a control command from the CPU 141. By doing so, the imager 13 moves relative to the specimen container 10 in the horizontal direction. Further, focusing is performed by moving the imager 13 in the vertical direction.

As indicated by arrows with dotted lines shown in FIG. 1, the driving mechanism 15 moves the illuminator 12 integrally with the imager 13 when the imager 13 is moved in the horizontal direction. Specifically, the illuminator 12 is arranged such that a center of emitted light substantially coincides with the optical axis of the imaging optical system. When the imager 13 moves in the horizontal direction, the illuminator 12 also moves in conjunction with the imager 13. By doing so, whenever the imager 13 moves relative to the specimen container 10, the center of light emitted from the illuminator 12 always positions on the optical axis of the imager 13. Consequently, the illuminating condition becomes constant regardless of which specimen container 10 is to be imaged, wherefore imaging conditions can be maintained to be satisfactory.

The image signal output from the imaging element 132 of the imager 13 is send to the controller 14. That is, the image signal is input to an AD converter (A/D) 143 provided in the controller 14 and converted into digital image data. The CPU 141 performs appropriate image processings based on the received image data.

The controller 14 further includes a memory 144 for temporarily storing image data and calculation result and a storage 145 for storing programs to be executed by the CPU 141 and data generated by the CPU 141. The memory 144 can be accessed from the CPU 144 at high speed, but has less storage capacity than the storage 145. The storage 145 includes a hard disk drive (HDD), for example, which has more storage capacity and less access speed than the memory 144. These can be used properly according to the purpose. The CPU 141 performs variable calculation processings described later by executing a control program stored in the storage 145.

Besides, the controller 14 is provided with an interface (IF) 142. The interface 142 has a function of receiving an operation input from a user and presenting information such as processing results to the user. The controller 14 also has a function of performing data exchange with an external apparatus connected via a communication line. To realize the user interface function, an input receiver 147 and a display 148 are connected to the interface 142. The input receiver 14 receives an operation input from the user. The display 148 for displaying the messages to the user, a processing result or the like

One embodiment of a cell counting method according to the invention executable using the imaging apparatus 1 configured as described above is described below. The imaging apparatus 1 has a function of automatically counting the number of cells constituting a cell mass as the biological specimen S by performing a cell counting process to be described later. Here, a process is described in which a fertilized egg (hereinafter, merely referred to as an "embryo") in an initial stage as the specimen S is imaged and the number of cells constituting this embryo is counted from those images. An embryo evaluating operation by a user (specifically, a doctor or embryologist) can be effectively supported based on data obtained by this process. For example, in the culture of an embryo for the purpose of a fertility treatment, the cell counting method of this embodiment can be applied for the purpose of obtaining knowledge for judging whether or not the culture is satisfactorily in progress. Note that an object to be processed is not limited to such an embryo, and this process is applicable to various cell masses in which a plurality of cells are closely in contact.

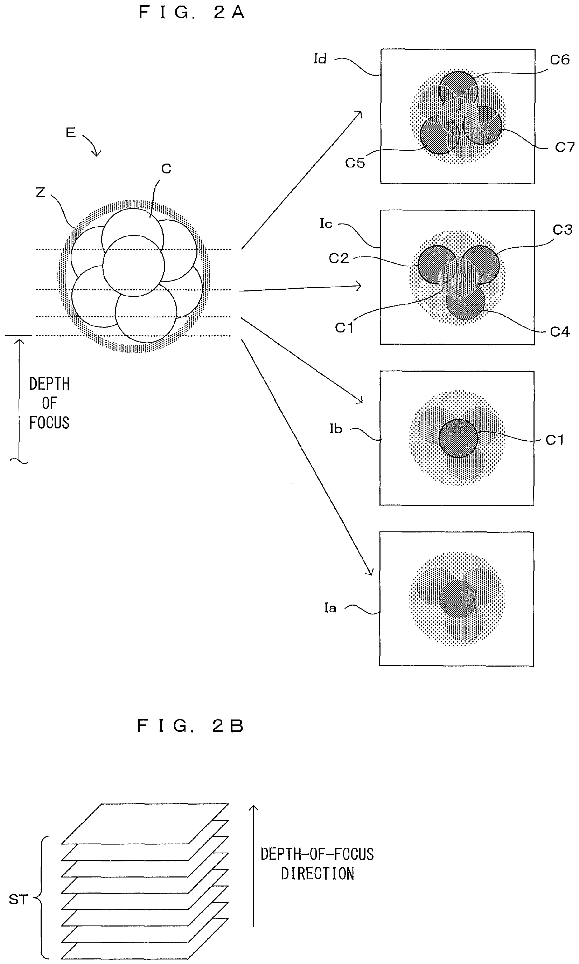

FIGS. 2A and 2B are diagrams schematically showing the structure of an embryo as a biological specimen in this embodiment. As already known, if an egg is fertilized, cleavage starts and a blastocyst is formed via a state called a morula. The cell counting method of this embodiment is suitable for counting the number of cells constituting an embryo, for example, until a morula stage immediately after fertilization.

FIG. 2A schematically shows the structure of the embryo in an initial stage (e.g. from a 4-cell stage to the morula stage). The embryo E has a substantially spherical outer shape. The surface of the embryo E is covered with a layer Z of a jelly glycoprotein called a zona pellucida, and a plurality of cells C produced by the cell division of the fertilized egg are included inside. In a state where the culture is satisfactorily in progress, the inside of the zona pellucida Z is taken up by relatively large cells C as shown in a left figure of FIG. 2A. As the cleavage proceeds, the number of the cells C increases.

If such an embryo E is imaged in a bright field while a depth of focus indicated by a dotted line in FIG. 2A is variously changed, a plurality of images schematically shown in right figures of FIG. 2A are obtained. In an image Ia having a smallest depth of focus when viewed from the imager 13 arranged below the specimen container 10, i.e. having a focus position positioned on a lowermost side, none of the cells C constituting the embryo E is focused and the image Ia is an unclear image.

In an image Ib in a state where the focus position is moved upward and, for example, the lowest cell C1 is focused, this cell C1 is clear. In an image Ic obtained by moving the focus position further upward, the cell C1 no longer in focus becomes unclear and the cells C2 to C4 at positions closer to the focus position appear as clearer images. If the cell C1 is nearly transparent, another cell located to the back of the cell C1 appears in the image through the cell C1. In an image Id obtained by moving the focus position further upward, the cells C1 to C4 no longer in focus become unclear. On the other hand, images of the upper cells C5 to C7 at positions closer to the focus position appear through the cells C1 to C4.

In this way, the same specimen is imaged in the same field of view a plurality of times while the depth of focus is changed and set in a multi-stage manner. Then, as shown in FIG. 2B, a set of a plurality of images having the same field of view and mutually different depths of focus can be obtained. This image set is called a multi-focus image stack. In each image in the image stack ST, the cells at positions close to the focus position, out of the cells constituting the cell mass (in this example, the embryo E), form clear images, whereas the images of the cells distant from the focus position are unclear. Further, the images of the cells to the back of the cells in front of the focus position (closer to the imager 13) appear through those front images. In the cell counting method of this embodiment, the number of the cells constituting the cell mass is counted, utilizing such characteristics of the image stack ST.

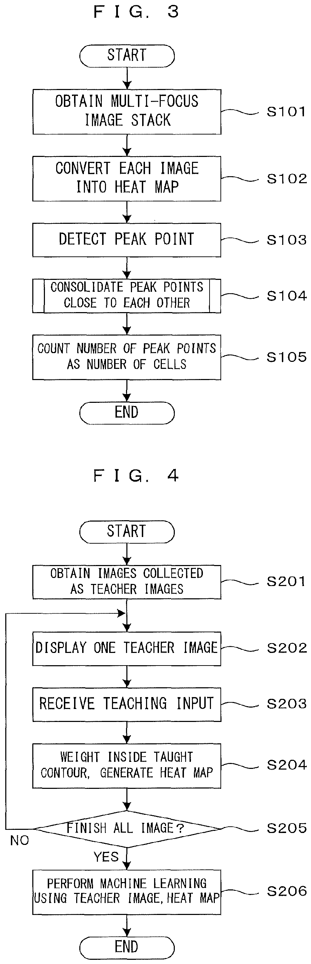

FIG. 3 is a flow chart summarizing the cell counting process of this embodiment. This process and each process described below are realized by the CPU 141 reading out and implementing a control program stored in advance in the storage 145 and causing each component of the apparatus to perform a predetermined operation. Note that these various processes can be realized also by a computer device having a general hardware configuration except the process of imaging the biological specimens. Thus, this embodiment may be realized by a combination of an imaging device having an imaging function and a computer device having an image processing function. Further, if an already captured image stack is present, an image processing using that image stack can be realized also by the computer device.

First, a multi-focus image stack ST is obtained by performing bright field imaging at mutually different depths of focus a plurality of times for the biological specimen S (here, the embryo E) as an object to be processed (Step S101). The multi-focus image stack ST may be newly obtained by the imaging of the imager 13 or image data obtained in advance by imaging and stored in an external storage or the like may be obtained from outside through an appropriate memory medium or an electric telecommunication line.

Each image in the image stack obtained in this way is converted into a heat map (Step S102). Specifically, if each of the plurality of images included in the image stack ST is input to a machine learning model constructed by machine learning in advance, the heat map corresponding to each image is obtained as an output. Although described in detail later, when an input image includes a clear image of a cell, the machine learning model outputs a heat map having a weight given thereto, the weight being larger at a position corresponding to the inside of the contour of this cell than outside the contour and increasing toward a central part of the cell.

In each of a plurality of the heat maps corresponding to the respective images, peak points having a larger weight than surroundings are detected (Step S103). As is understood from the aforementioned characteristics of the heat map, there is a high probability that the weight peak points represent center positions of the individual cells. Therefore, there can be said to be a certain correlation between the number of peak points detected from the respective image and the number of cells constituting the cell mass.

However, one cell may appear over a plurality of images having different depths of focus. Then, the same cell is detected as the peak point in a plurality of images. It is necessary to avoid the repeated counting of these. For this purpose, out of the peak points detected in a plurality of images, those close to each other and having a high possibility of being derived from the same cell are consolidated into one (Step S 104). A method for that is described later.

The peak points after being consolidated in this way are thought to represent a center position of one cell. Accordingly, the peak points are counted and that count result is regarded as the number of the cells (Step S 105). In this way, it is avoided to repeatedly count one cell appearing in a plurality of images, and the cells can be accurately automatically counted.

Next, a process of generating the machine learning model for outputting the heat map is described. As described above, in the heat map, a larger weight is given toward a central part of a cell for a clear image of the cell included in an original image. On the other hand, if the image includes no cell image or the cell image is unclear, the weight is zero in an entire region.

As just described, the cell image is converted into a heat map having such a weight that is largest in a region which is highly probably a central part of the cell and decreases with distance from the center part in the surroundings. This conversion can be realized by using the machine learning model constructed as follows by supervised machine learning.

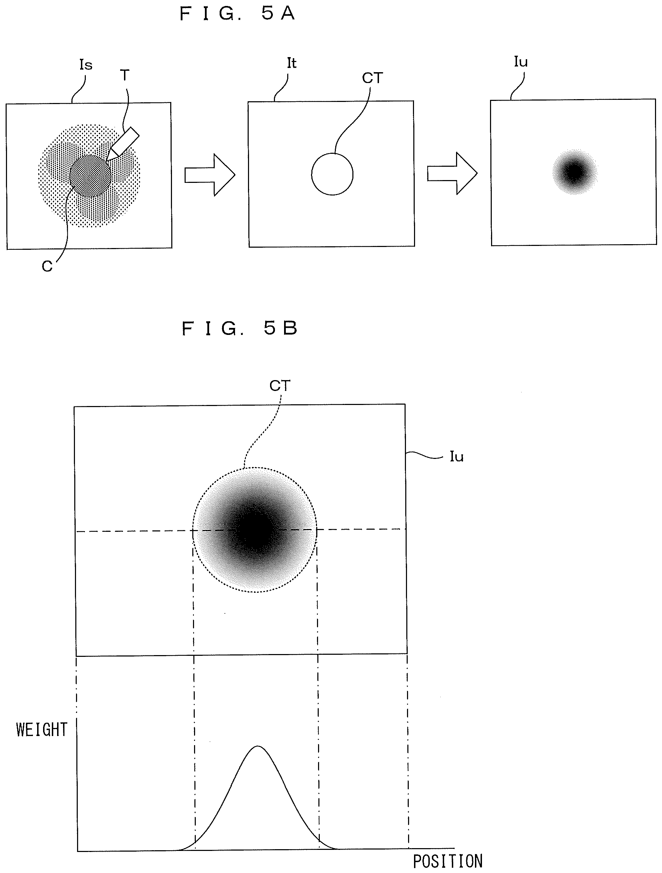

FIG. 4 is a flow chart showing a process of constructing the machine learning model. Further, FIGS. 5A and 5B are charts illustrating a process of generating the heat map. First, images collected as teacher images are obtained (Step S201). The teacher images are a plurality of images including images of cells captured in an in-focus condition, particularly having clear contours. For example, images showing clearly appearing cells and selected by a skilled person, out of a plurality of images obtained by the multi-focus bright field imaging of the embryo E or the same type of the specimen S as an object to be processed, can be set as the teacher images.

It is preferred to collect as many teacher images as possible. Further, images of a plurality of specimens S are preferably collected to deal with individual differences of cells. Further, to improve learning accuracy by increasing the number of the teacher images, images interpolated in a depth-of-focus direction using two images included in a multi-focus image stack and having depths of focus different by one level may be generated as teacher images.

Image data of the teacher images thus collected is, for example, saved in the storage 145. Out of those, one image is displayed on the display unit 148 (Step S202), and a teaching input from the user for the displayed image is received (Step S203). Note that a plurality of teacher images may be displayed on one screen.

Teaching is input to specify a clear cell region included in each teacher image. For example, as shown in FIG. 5A, the user can input teaching by tracing a clear contour of a cell C included in a displayed image Is using an appropriate drawing tool T. Alternatively, teaching may be input by superimposing and arranging a circle or ellipse substantially matching a peripheral edge part of the cell C on the image. The user teaches the region of the cell in the image by an appropriate method in this way, whereby each teacher image is labeled.

The teacher images may include images not including a clear cell image like the image Ia shown in FIG. 2A. If the teacher image does not include the cell image or the cell image is unclear, the teaching of the contour is not provided. This is technically equivalent to giving a label meaning that no clear cell image is present to this image. Further, like the image Ic shown in FIG. 2A, a plurality of clear cell images may be included in one teacher image. In that case, it is desirable to teach the contour of each cell and distinguish those cells to be handled as individual ones.

A label image It is generated, using a region surrounded by the thus taught contour CT as a teacher label. By further weighting the inside of the contour, a heat map image Iu is generated (Step S204). A teaching input is received for all the collected teacher images (Step S205).

As shown in FIG. 5B, the heat map image Iu is an image having a weight given thereto, the weight being larger inside the taught contour CT than outside and increasing toward a central part of the region surrounded by the contour CT. Note that the heat map image Iu shown here is expressed as an image having a higher density with increase in the weight, i.e. having a low luminance as an image. However, contrary to this, the heat map image Iu may be expressed to have a higher luminance to be nearly white with increase in the weight.

For example, a centroid of the region surrounded by the contour CT can be obtained and weighting can be performed along a two-dimensional Gaussian distribution centered on the centroid. Further, weighting may be performed by a distance map for giving a weight corresponding to a distance from the contour CT to the inside of the region surrounded by the contour CT. In this way, the heat map image Iu can be generated by applying various weighting methods to increase a weight toward the central part inside the region surrounded by the contour CT.

As shown in FIG. 5B, the weight needs not necessarily be zero at the position of the contour CT. In short, weighting may be performed such that the region surrounded by the contour CT and the outside region can be distinguished and the weight increases toward a center of the internal region. In this sense, the teaching of the contour CT may not strictly designate the contour of the cell in the image. Further, to clearly distinguish the inside and outside of the cell, a certain offset value may be added to the weight given to the inside of the contour CT.

A magnitude of the weight given to each point on the heat map relatively represents a probability that this point corresponds to the center of the cell. That is, a position where the weight is maximum in the heat map represents the center position of the cell. The center of the cell mentioned here is a point most distant from other cells and the like if this cell is in contact with the other cells and the like, and is not directly related to a structure in this cell, e.g. the position of a cell nucleus and the like.

Subsequently, machine learning is performed using, sets of each of the plurality of teacher images Is and the corresponding heat map image Iu as teacher data (Step S206). A machine learning algorithm can be appropriately selected from various methods known as supervised machine learning methods and used. For example, a convolutional neural network method can be used.

By performing machine learning using the teacher image Is as an input image and the corresponding heat map image Iu as a ground truth image, the machine learning model is constructed. The thus constructed machine learning model has acquired a function of outputting, for a newly given image of a cell, a heat map represented by a probability distribution of the center position of the cell estimated from this image. That is, the heat map image output from the learned machine learning model provides information for estimating the center position of the cell in the input image.

Specifically, a peak point at which the magnitude of the weight is maximized as compared to surrounding regions may be searched and detected in the output heat map image. This peak point can be said to represent a position where a probability of being the center of the cell is highest. A substantial peak point does not appear in an image including no clear cell image. The detection of the peak point (Step S103) can be, for example, performed as follows.

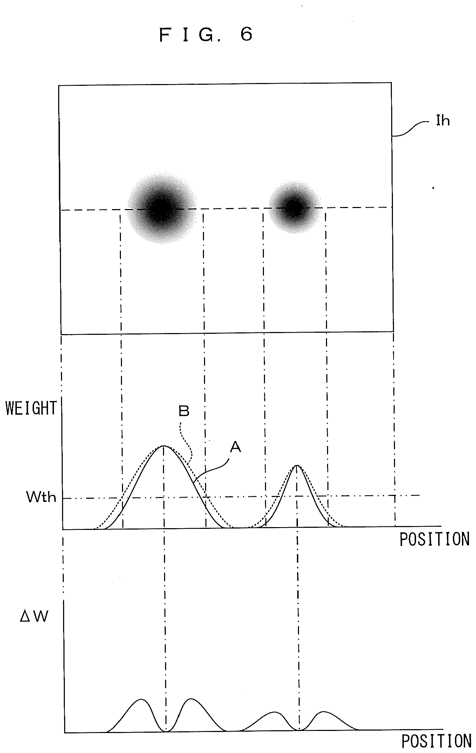

FIG. 6 is a diagram showing an example of the method for detecting the peak points. Note that, to facilitate understanding, a relationship of the position and the weight in the heat map image is shown only in a one-dimensional direction. However, an actual weight is distributed in a two-dimensional image plane.

For a heat map image Ih outputted by the learned machine learning model shown in an upper part of FIG. 6, a pre-processing is performed to exclude regions needed not be considered in the following process. For example, regions where a cell is clearly not present such as the outside of a container, regions significantly smaller than an assumed cell size and clearly regarded not as a cell and the like are erased by binarization or masking. After that, filtering is performed to remove finer irregularities caused by image noise or the like. For example, moving-average filtering can be applied. Then, a blur image is obtained which has a smooth probability distribution curve (actually a curved surface corresponding to a two-dimension image) like a curve A shown in a middle part of FIG. 6. If maximum value filtering is performed for this blur image, a maximum value image is obtained which looks as if the probability distribution curve were caused to bulge in a lateral direction like a curve B. A value does not change at the peak point having a larger weight than the surroundings.

By taking a difference between the maximum value image and the blur image, a weight difference ΔW is zero in a region with a weight of zero by being distant from the cell and at the peak points as shown in a lower part of FIG. 6. By detecting points where the difference is zero, the peak points can be detected. To distinguish the regions distant from the cells and the peak points, an appropriate threshold Wth may be set for the value of the weight as shown in the middle part of FIG. 6 and the points having a larger weight than the threshold Wth and having the difference ΔW of zero may be detected as the peak points.

The peak point detected in this way can be thought to represent the center position of the cell relatively clearly appearing in each image of the multi-focus image stack ST. However, when one cell appears over a plurality of images, the peak point representing the same cell is detected in the plurality of images. To avoid the repeated counting due to this, the peak points thought to be derived from the same cell are associated with each other in this embodiment. Those peak points are consolidated and regarded as one peak (Step S104).

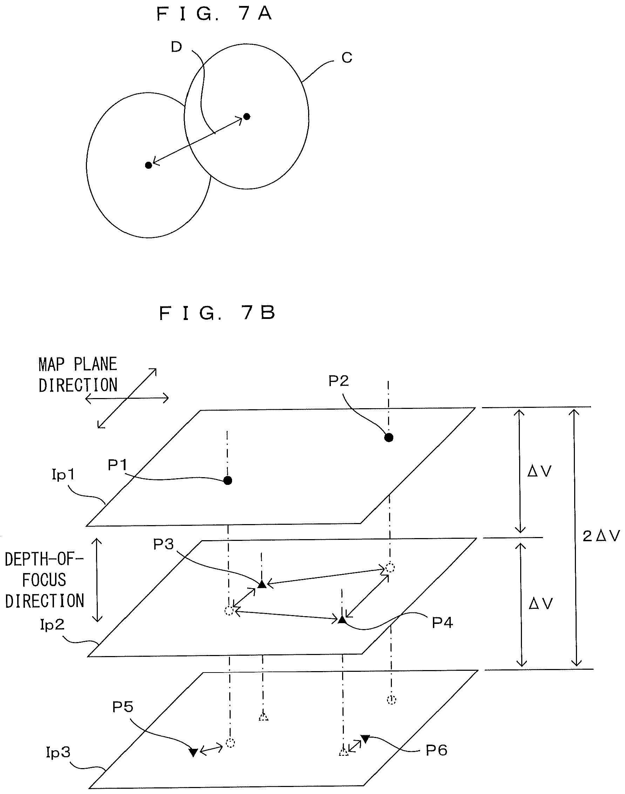

FIGS. 7A and 7B are diagrams showing a process of consolidating the peak points. In a cell mass formed by aggregating the same type of cells, the sizes of the cells are uniform to a certain extent. Particularly, an embryo in an initial stage of development and in a good condition is formed by cells having fairly uniform sizes. Thus, as shown in FIG. 7A, a center-to-center distance D of cells C, C in contact with each other is thought not to be smaller than a minimum distance determined according to the sizes of the cells C. Even if the sizes of the cells vary, a similar way of thinking can be applied if a minimum distance derived from the sizes of relatively small cells is used.

The peak point detected from the heat map image Ih corresponds to the center position of the cell. Accordingly, if peak points detected from a plurality of heat map images are derived from mutually different cells, distances therebetween can be said not to be smaller than the above minimum distance. From this, it is reasonable to think that, out of the detected peak points, those separated from each other by distances sufficiently smaller than a predetermined value assumed from the cell sizes in an actual space are derived from the same cell. The detected peak points are consolidated based on this way of thinking.

A more specific case example is described with reference to FIG. 7B. An image in which only the peak points detected from the heat map image Ih are extracted and arranged on a map plane is called a peak image. It is assumed that three peak images Ip1, Ip2 and Ip3 were obtained from three images captured while the depth of focus is made different by ΔV It is assumed that, out of these, peak points P1, P2 were detected from the peak image Ip1, peak points P3, P4 were detected from the peak image Ip2 and peak points P5, P6 were detected from the peak image Ip3. Note that, to clearly show the peak points on each peak image, a distance in the depth-of-focus direction is shown longer than a distance in a direction along the map plane (hereinafter, referred to as a "map plane direction") in FIG. 7B.

The peak point P1 on the peak image Ip1 and the peak point P3 on the peak image Ip2 are at relatively close positions in the map plane direction. Further, a distance between these peak points in the depth-of-focus direction is ΔV. The peak points P1, P3, which are peak points on the different peak images and at the relatively close positions in the map plane direction and the depth-of-focus direction, have a high possibility of being derived from the same cell. Although described in detail later, a state "close" mentioned here is a concept judged from a relationship with an assumed cell size.

On the other hand, there is a relatively large distance in the map plane direction between the peak point P1 and peak point P2, between the peak point P3 and the peak point P4, between the peak point P2 and the peak points P3, P4 and between the peak point P1 and the peak point P4. Therefore, these peak points are thought to be derived from mutually different cells.

Further, the peak point P1 on the peak image Ip1 and the peak point P5 on the peak image Ip3 are at relatively close positions in the map plane direction, but a distance in the depth-of-focus direction is 2ΔV and these peak points are relatively largely separated. Accordingly, these peak points P1, P5 are thought to be derived from mutually different cells. Similarly, the peak point P4 on the peak image Ip2 and the peak point P6 on the peak image Ip3 are close in each of the map plane direction and the depth-of-focus direction and through to be derived from the same cell.

As just described, the peak points in a proximate relationship in both the map plane direction and the depth-of-focus direction, i.e. the peak points P1 and P3 and the peak points P4 and P6 are respectively regarded as derived from the same cell and associated with each other. On the other hand, the other peak points P2, P5 not in a proximate relationship with the other peak points in at least one of the map plane direction and the depth-of-focus direction are regarded as isolated peak points derived from mutually different cells.

The peak points associated as those proximate to each other are consolidated and handled as one group, whereas each of the isolated peak points is handled as one group, and the number of the groups after consolidation is counted (4 in this example). In this way, the number of the cells can be obtained while repetition due to the appearance of one cell in a plurality of images is avoided. In the teacher images and the machine learning model constructed by proper teaching for the teacher images, the number of the cells can be automatically calculated thereafter without requiring human subjective judgment.

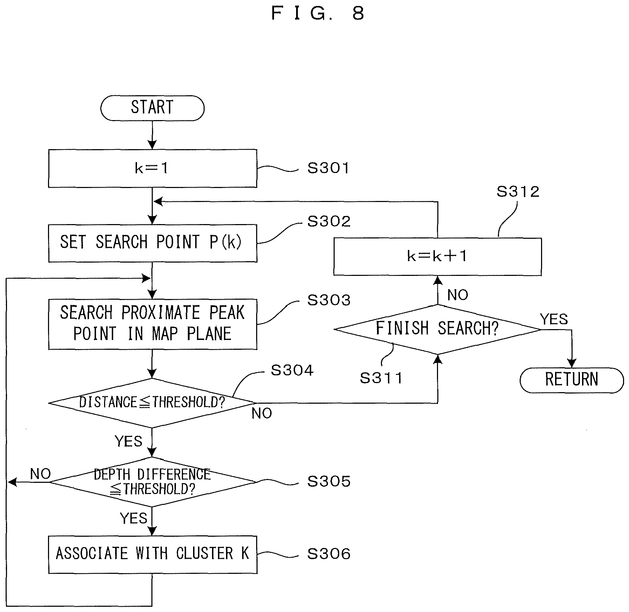

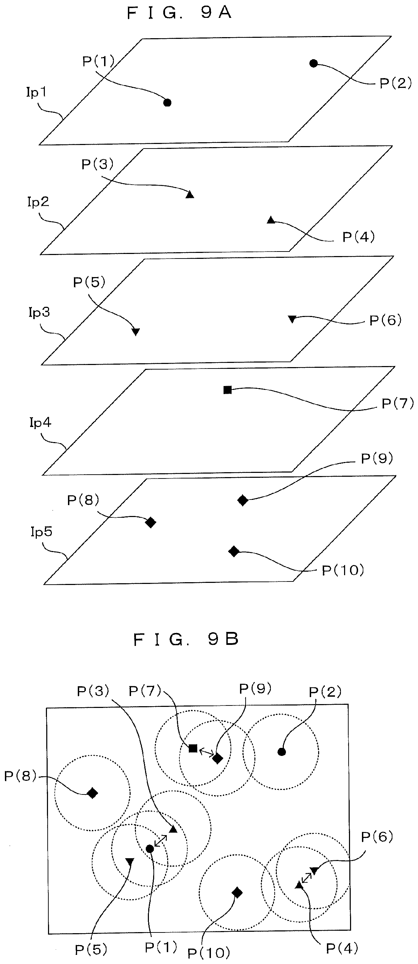

FIG. 8 is a flow chart showing a first aspect of a proximate peak consolidation process. Further, FIGS. 9A and 9B are diagrams schematically showing an example of this proximate peak consolidation process. Here, FIG. 9A is a diagram showing the positions of peak points P(1) to P(10) of five peak images Ip1 to Ip5. Further, FIG. 9B is a diagram in which those peak points are arranged on a map plane and corresponds to an image in which the peak images Ip1 to Ip5 are superimposed.

First, an internal parameter k is set to 1 (Step S301) and a search point P(k) is set (Step S302). At this point of time, the peak point P(1) is set as the search point. Then, in the map plane shown in FIG. 9B, a proximate peak point having a shortest distance from the search point P(1) is searched (Step S303). In an example shown in FIG. 9B, the peak point P5 closest to the search point P(1) is detected as the proximate peak point in the map plane.

It is assumed that a distance between the search point P(k) and the proximate peak point in the map plane is equal to or less than a first threshold (YES in Step S304) and a distance therebetween in the depth-of-focus direction is equal to or less than a second threshold (YES in Step S305). Then, the proximate peak point is a peak point detected near the search point P(k) in the actual space and it is reasonable to think that the both points are derived from the same cell. Accordingly, this proximate peak point is associated with the search point P(k) by being handled as belonging to a cluster k including the search point P(k).

The first threshold applied to the distance in the map plane direction and the second threshold applied to the distance in the depth-of-focus direction are values which can be appropriately determined according to the cell size. Specifically, these thresholds can be values corresponding to a minimum value assumed as a distance between centers of adjacent cells. The map plane direction and the depth-of-focus direction are expedient directions determined by a situation at the time of multi-focus imaging. The cells do not inherently have anisotropy for these directions. Therefore, there is, in principle, no need to set the first and second thresholds to different values. That is, the first and second thresholds can be the same value.

Realistically, a distance resolution in the map plane can be, for example, reduced to about a pixel size. Thus, the first threshold can be freely set according to the cell size. On the other hand, a resolution in the depth-of-focus direction depends on a depth of field of the imaging optical system including the objective lens 131 and the setting of a focus position adjustment pitch during imaging. Further, an actual focus position is affected by refraction caused by the cells and the culture medium. From these, the second threshold is not necessarily determined similarly to the first threshold. Under a condition that the depth of field of the imaging optical system is sufficiently small, the second threshold can be determined as follows, for example, based on a relationship of pitch setting during imaging and the cell size.

For the purpose of multi-focus imaging to image while each of cells constituting a cell mass is successively focused, it is thought to set the focus position adjustment pitch equal to or slightly smaller than the cell size. Then, a range in which a cell clearly imaged in one image possibly appears also in other images may be thought to be up to adjacent images having the focus positions set to be different by one level. That is, the focus position adjustment pitch may be set as the second threshold. Similarly, if the focus position adjustment pitch is, for example, about half the cell size, it is sufficient to consider up to images having depths of focus different by two levels. That is, twice the focus position adjustment pitch may be set as the second threshold.

Of course, even if the second threshold is set similarly to the first threshold and, in Step S305, the distance between the peaks in the depth-of-focus direction is calculated based on the focus position adjustment pitch and compared to the second threshold every time, it is technically equivalent. However, the process is simple and more realistic if depth of focus differences up to which level are judged as "proximate" is determined in advance.

In an example shown in FIG. 9B, the second threshold is assumed to be set to the focus position adjustment pitch. Then, the peak P(5) detected as the most proximate peak point of the search point P(1) has the depth of focus during imaging, which is different by 2 levels between the peak images Ip1, Ip3 in which those points are detected. That is, the distance in the depth-of-field direction exceeds the second threshold. Therefore, the search point P(1) and the proximate peak point P(5) are not associated.

If NO in Step S304, i.e. if the detected proximate peak point is at a distance larger than the first threshold from the search point P(k), no other peak point to be possibly associated with this search point P(k) exists. Accordingly, the internal parameter k is increased by 1, return is made to Step S302 (Step S312), and a similar process is performed using another peak point, e.g. P(2), as a new search point. If this peak point is associated with another peak point, it may be excluded from the search. If the search has been finished for all the peak points, the process is finished (Step S311).

Next, a case is considered where NO in Step S305, i.e. the distance between the detected proximate peak point and the search point P(k) is equal to or less than the first threshold in the map plane, but exceeds the second threshold in the depth-of-field direction. In this case, another peak point close to the search point P(k) next to the current proximate peak point is searched in the map plane (Step S303). The process in and after Step S304 is performed also for the thus detected peak point.

By doing so, even if there are a plurality of peak points near the search point P(k) in the map plane, all of those can be evaluated. Dotted lines in FIG. 9B represent circles having a center at each peak point and a radius set to the first threshold. If one of the respective peak points P(k) is a search point, other peak points located in the circle centered on this peak point are successively searched and distances thereof in the depth-of-field direction are evaluated, whereby peak consolidation proceeds.

In the example shown in FIG. 9B, the peak point close to the search point P(1) next to the peak point P(5) is the peak point P(3). The focus position setting during imaging is different by one level between the peak image Ip1 including the search point P(1) and the peak image Ip2 including the peak point P(3). Therefore, a distance in the depth-of-field direction between the search point P(1) and the other peak point P(3) is judged to be equal to or less than the second threshold, and the both points are associated as derived from the same cell. At the time of counting, two peak points are counted as one cell.

Similarly, the distances between the respective peak points in the map plane direction and the depth-of-field direction are evaluated. In this way, the peak points P(4) and P(6) are associated and the peak points P(7) and P(9) are associated, and each of these pairs is counted as one cell. On the other hand, each of the isolated peak points P(2), P(5), P(8) and P(10) not associated with other peak points is counted as one cell. Therefore, in the example shown in FIG. 9B, the number of the peak points is 10, but 7 is obtained as a count result of the cell number since the peak points thought to be derived from the same cells are consolidated. In this way, it is avoided that one cell is repeatedly counted.

Note that the number of the peak points detected from each image in the multi-focus image stack ST varies. For example, it is also possible that no peak point is included in one image. Further, a situation where a plurality of detected peak points are not associated with each other at all possibly occurs. The above method can also deal with such cases and can accurately count the number of the cells.

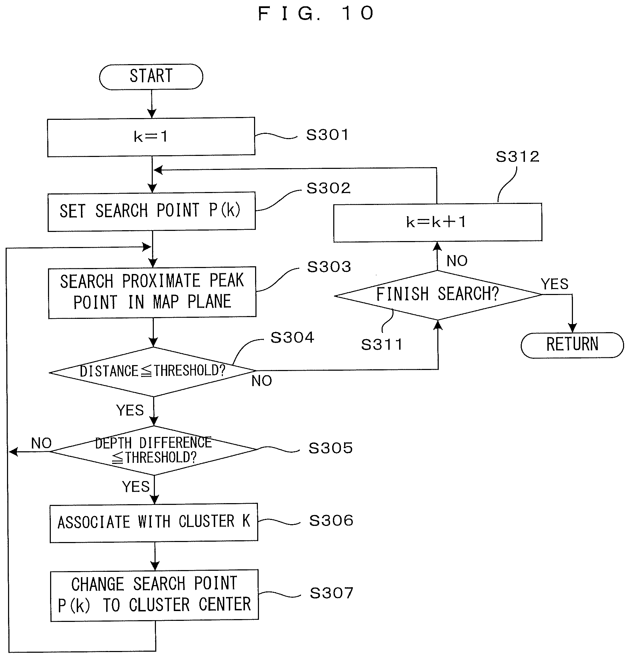

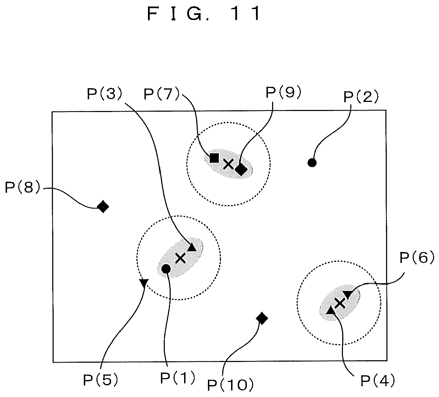

FIG. 10 is a flow chart showing a second aspect of the proximate peak consolidation process. Further, FIG. 11 is a diagram schematically showing an example of this proximate peak consolidation process. The second aspect differs from the first aspect shown in FIG. 8 in that Step S307 is provided after Step S306. Since the contents of processing other than this are the same, processing steps having the same contents of processing are denoted by the same numbers and not described.

In this aspect, when the peak points are associated with each other in Step S306, the center of the search point P(k) is changed and set at a center position of a cluster formed by the associated peak points. A mark "×" shown in FIG. 11 represents a cluster center. Thereafter, the proximate peak point is searched in the map plane based on a comparison of a distance from the cluster center and the first threshold. Dotted lines of FIG. 11 represent circles centered at the cluster centers and having a radius set to the first threshold.

The peak point specified by the above method from each image obtained by multi-focus imaging indicates a center position of an image of a cell in this image. However, the detected position does not necessarily match a center of the cell in the actual space. By setting the cluster position of the cluster composed of the peak points associated with each other as the search point, the search can be performed from a position closer to the center of the cell in the actual space. Similarly, a distance may be evaluated also in the depth-of-field direction on the basis of the center position of the cluster.

In the first aspect shown in FIG. 8, the position of each peak point is set as a center at the time of the search. Thus, particularly if the cells are densely present or the like, the adjacent peak points are possibly associated in a chain-like manner to form a large cluster. Then, a cluster of a size largely exceeding the cell size is possibly regarded as one cell. By setting the center position of the cluster as the search point, such a problem can also be avoided.

Note that, realistically speaking, if each of the focus position adjustment pitch, the first threshold and the second threshold is properly set in accordance with the assumed cell size, the problem described above does not occur and it is thought that there is no large difference between the results in the two process aspects.

As described above, in this embodiment, each of the plurality of images obtained by the multi-focus imaging of the cell mass is converted into the heat map with a weight increasing toward the center of cell in each image, using the machine learning model learned in advance. Then, the peak point having a maximum weight in the heat map is detected, the distance between the peak points is evaluated in each of the map plane direction and the depth-of-focus direction, and the peak points having a high probability of being derived from the same cell are associated with each other and consolidated. The distance is evaluated by a comparison to the threshold determined from the assumed cell size. Thus, it is avoided that the plurality of peak points derived from the same cell are repeatedly counted, and the number of the cells constituting the cell mass can be accurately counted by counting the number of the peaks.

The machine learning model is constructed by supervised learning based on the teacher images including clear images of the cells and the ground truth images in each of which the heat map is disposed inside the taught contour in the teacher image. Thus, the center position of the cell in the image can be accurately specified using the learned model. Then, evaluation is performed based on the distance between the centers of the cells specified from the contours of the cells. By doing so, the peak points derived from the same cells and the peak points derived from different cells can be distinguished without being affected by the textures inside the cells, unlike the conventional technique utilizing, for example, stained cell nuclei.

Note that the invention is not limited to the embodiment described above and various changes other than the aforementioned ones can be made without departing from the gist of the invention. For example, the imaging apparatus 1 of the above embodiment has itself the imaging function of obtaining the multi-focus image stack and the image processing function of analyzing the images and performing the cell counting process. However, the cell counting method according to the invention can also be performed by a computer device which has no imaging function itself and obtains an image stack obtained by imaging in another apparatus having an imaging function. To enable this, the invention may be carried out as a software (computer program) for causing the computer device to perform each processing step of the above process.

Such a program can be distributed, for example, in the form of downloading via an electric telecommunication line such as the Internet. Further, the program can also be distributed by distributing a computer-readable recording medium recording this program. Further, by causing, for example, an existing microscope imaging apparatus to read this program via an interface, the invention can be carried out by this apparatus.

Further, in the above embodiment, the construction of the machine learning model based on the collected teacher images and the cell counting process utilizing the constructed machine learning model are performed by the same apparatus. However, these may be performed by different apparatuses. For example, if the machine learning model is constructed using a computer device having a high computing power and the result is implemented into an imaging apparatus, even the imaging apparatus having a limited computing power can perform the imaging and the cell counting process.

Further, in the peak consolidation process of the above embodiment, the peak-to-peak distance is individually evaluated in each of the map plane direction and the depth-of-focus direction. Instead of this, the peak-to-peak distance may be three-dimensionally evaluated based on whether or not the peaks are located within a sphere centered on the search point and having a predetermined radius. The peaks can be consolidated, for example, by applying an X-means method or clustering method.

Further, in the above embodiment, the embryo (fertilized egg) in the initial stage of development is used as the cell mass as an object to be processed of the invention. However, a cell mass as an object of the invention is not limited to this. Particularly, the invention is suitably applicable for the purpose of automatically counting the number of cells constituting a cell mass composed of a plurality of the cells having uniform sizes.

As the specific embodiment has been illustrated and described above, out of a plurality of peaks detected from mutually different heat maps and having a mutual distance in a direction along a map plane smaller than a first threshold, the peaks which have a difference of the depth of focus in imaging the images corresponding to the heat maps smaller than a second threshold can be associated with each other in the cell counting method according to the invention. Here, the first and second thresholds may be the same value.

Further, the teacher image is preferably an image obtained by imaging a same type of cell mass as the cell mass as an object of counting. By doing so, the cells can be accurately extracted from the images by effectively reflecting characteristics of the cells included in the cell mass serving as the object of counting on a learning result.

In this case, the teacher images preferably include an image focused on a peripheral edge part of at least one cell. By performing machine learning using the images having clear contours of the cells as the teacher images, a heat map properly indicating a center position of the cell can be generated for the image similarly having a clear contour in the image stack.

Further, the teacher images may be selected from a plurality of images of the cell mass obtained by bright-field imaging at mutually different depths of focus. Each of the images obtained by imaging the cell mass including a multitude of the cells at various depths of focus possibly includes a clear image of the cell located at a position corresponding to the depth of focus when this image was captured. By using the images including such images of the cells as the teacher images, the shapes, colors and the like of the cells in the cell mass can be effectively reflected on the learning result.

Further, in a construction method of the machine learning model for cell counting according to the invention, for example, for each of the obtained teacher images, a teaching input teaching the contour of the cell in the teacher image is received, and a weight larger inside a region surrounded by the taught contour than outside that region and increasing toward a central part of this region can be given in the heat map. By first specifying the contour of the cell and giving a proper weight distribution inside the contour, it is possible to construct the machine learning model capable of precisely indicating the center position of the cell without being affected by the texture inside the cell.

The machine learning model may have a function of estimating the center of the cell with such accuracy as to be able to evaluate a distance to another cell. Thus, an arbitrary distribution to be larger inside the cell than outside the cell and increase toward the central part of the cell may be given for weighting in the heat map. A method in accordance with a two-dimensional Gaussian distribution centered on the center of the cell can be, for example, used as a typical method.

Although the invention has been described with reference to specific embodiments, this description is not meant to be construed in a limiting sense. Various modifications of the disclosed embodiment, as well as other embodiments of the present invention, will become apparent to persons skilled in the art upon reference to the description of the invention. It is therefore contemplated that the appended claims will cover any such modifications or embodiments as fall within the true scope of the invention.

### [Industrial Applicability]

This invention enables the number of cells in a cell mass as an aggregate of a plurality of cells to be automatically and accurately counted. For example, in the field of assisted reproductive medicine, an operation of evaluating a state of a cultured embryo (fertilized egg) can be effectively supported.

### [Reference Signs List]

- 1: imaging apparatus
- 10: specimen container
- 13: imager
- 14: controller
- C: cell
- E: embryo (cell mass)
- Ih, Iu: heat map
- Is: teacher image
- S: sample
- ST: image stack

## Claims

1. A cell counting method for counting number of cells constituting a cell mass, the cell counting method comprising:
obtaining an image stack including a plurality of images of the cell mass obtained by bright-field imaging at mutually different depths of focus;
generating a plurality of heat maps respectively corresponding to the plurality of images using a machine learning model;
detecting a peak from each of the heat maps;
associating the peaks belonging to mutually different ones of the heat maps and having a distance in a direction along a map plane and a distance in a direction of the depth of focus, the distances being respectively smaller than predetermined values determined in advance, with each other; and
counting number of the peaks, regarding a plurality of the associated peaks as one peak, wherein
the machine learning model is constructed by machine learning, using sets of a teacher image and a ground truth image as teacher data, the teacher image being a bright-field image of a cell, the ground truth image being a heat map to which a weight larger inside a contour of the cell focused in the teacher image than outside the contour and increasing toward a central part of the cell is given.

2. The cell counting method according to claim 1, wherein, out of a plurality of peaks detected from mutually different heat maps and having a mutual distance in a direction along a map plane smaller than a first threshold, the peaks which have a difference of the depth of focus in imaging the images corresponding to the heat maps smaller than a second threshold are associated with each other.

3. The cell counting method according to claim 2, wherein the first threshold and the second threshold are same value.

4. The cell counting method according to any one of claims 1 through 3, wherein the teacher image is an image obtained by imaging a same type of cell mass as the cell mass as an object of counting.

5. A computer program, causing a computer to perform each step of the cell counting method according to any one of claims 1 through 4.

6. A machine learning model construction method for cell counting, the machine learning model construction method comprising:
obtaining a plurality of teacher images which are bright-field images of a cell mass;
generating a ground truth image for each of the teacher images which is a pseudo heat map to which a weight is given, the weight being larger inside a contour of a cell focused in the teacher image than outside the contour and increasing toward a central part of the cell; and
constructing a machine learning model by performing machine learning, using sets of the teacher image and the ground truth image as teacher data.

7. The machine learning model construction method according to claim 6, wherein the teacher images include an image focused on a peripheral edge part of at least one cell.

8. The machine learning model construction method according to claim 6 or 7, wherein the teacher images are selected from a plurality of images of the cell mass obtained by bright-field imaging at mutually different depths of focus.

9. The machine learning model construction method according to any one of claims 6 through 8, wherein,
for each of the teacher images, a teaching input teaching the contour of the cell in the teacher image is received, and
a weight larger inside a region surrounded by the contour which is taught than outside that region and increasing toward a central part of this region is given in the heat map.

10. The machine learning model construction method according to any one of claims 6 through 9, wherein the weight in accordance with a two-dimensional Gaussian distribution centered on a center of the cell is given in the heat map.

11. A computer program, causing a computer to perform each step of the machine learning model construction method according to any one of claims 6 through 10.

12. A computer-readable recording medium, non-transitorily recording the computer program according to claim 5 or 11.
